# EUROPÄISCHE PATENTSCHRIFT

(11) **EP 0 455 172 B1**
(45) Veröffentlichungstag und Bekanntmachung des Hinweises auf die Patenterteilung: **30.08.1995**
(21) Anmeldenummer: 91106861.7
(22) Anmeldetag: 26.04.1991
(51) Int. Cl.: A61B 19/02, B65F 1/16, B65D 1/20, B65D 43/00, B65D 81/00, B65F 1/02

(54) **Einweg-Sammel- und Transportbehälter**
One-time use collection and transport container
Récipient de collecte et de transport à usage unique

(30) Priorität: 30.04.1990 DE 4013927
(43) Veröffentlichungstag der Anmeldung: 06.11.1991
(73) Patentinhaber: Rigling GmbH, D-75382 Althengstett (DE)
(72) Erfinder: Rigling, Heinz, D-75382 Althengstett (DE)
(74) Vertreter: Liesegang, Roland, Dr.-Ing.

(56) Entgegenhaltungen:
- EP-A- 0 049 430
- EP-A- 0 343 680
- US-A- 3 977 563

## Beschreibung

Die Erfindung betrifft einen Einweg-Sammel- und Transportbehälter zur Entsorgung von klinischem Abfall und/oder anderem medizinischen Sondermüll gemäß dem Oberbegriff des Anspruchs 1 (EP-A-0 343 680).

Behälter dieser Art werden insbesondere zur Entsorgung in Krankenhäusern eingesetzt (EP-A-0 049 430). Die Behälter werden ineinander gestapelt angeliefert. Nach dem Befüllen werden die Behälter durch Aufkleben eines separaten Deckels oder durch Zuklappen eines angeformten Deckels luftdicht verschlossen und danach entsorgt, z.B. verbrannt.

Der Erfindung liegt die Aufgabe zugrunde, einen Behälter der oben beschriebenen Art zu schaffen, der eine platzsparende Lagerung und Anlieferung vor dem Gebrauch, einfache Handhabung und gute Dichtung sowohl während der Gebrauchszeit mit wiederholtem Einfüllen, eine absolut gasdichte, das Austreten von Keimen verhindernde Dichtung jedenfalls nach der endgültigen Befüllung sowie eine ausreichende Stabilität des Deckels zur Übereinanderstapelung mehrerer befüllter Behälter gewährleistet.

Zur Lösung dieser Aufgabe dient Anspruch 1.

Ein Behälter nach der Erfindung wird getrennt von äußerem und innerem Deckel gefertigt, so daß er zur Zwischenlagerung und Anlieferung platzsparend ineinander stapelbar ist. Zum Gebrauch wird der äußere Deckel mit dem eingesteckten oder eingeschraubten inneren Deckel in die Behälteröffnung eingedrückt. Bei der normalen Nutzung reicht die Beschickungsöffnung zur Aufnahme des zu entsorgenden Materials aus. Zum Freigeben und provisorischem Verschließen dieser Beschickungsöffung ist der innere Deckel durch die Schraub- oder Steckverbindung mit der Beschickungsöffnung im Gebrauch besonders einfach handhabbar. Lediglich zur Aufnahme von sperrigen Gegenständen kann im Bedarfsfalle auch der äußere Deckel entfernt werden, so daß eine sehr viel größere Beschickungsöffnung zur Verfügung steht.

Die Abdichtung zwischen dem äußeren Deckel und dem oberen Behälterrand oder - bei der Ausführung mit Steckdeckel - zwischen Beschickungsöffnung und Steckdeckel wird durch die dichtend ausgebildete Konturierung dieser beiden Teile, ggf. unter Zwischenfügen eines Dichtmaterials oder Dichtringes erzielt, was zu einer gasdichten, keimfreien Dichtung führt.

Bei einer Ausführung mit Schraubdeckel weist dieser Gewindeabschnitte auf, die unter entsprechende Gewindeabschnitte in der Beschickungsöffnung greifen, wobei nach Art einer Bajonett-Verbindung die Abstände zwischen zwei in Umfangsrichtung aufeinander folgenden Gewindeabschnitten in der Beschickungsöffnung zum Durchlassen der Gewindeabschnitte am Schraubdeckel bemessen sind. Hierbei wird in voll eingeschraubtem Zustand eine Verrasterung der Gewindeabschnitte dadurch erzielt, daß jeweils eine Rastnase am inneren Ende des Gewindeabschnittes des Schraubdeckels mit einer Rastaussparung am inneren Ende jedes Gewindeabschnittes der Beschickungsöffnung in voll eingeschraubtem Zustand des Schraubdeckels zusammenwirkt.

Eine gasdichte Abdichtung der Beschickungsöffnung wird in voll eingeschraubtem Zustand des Schraubdeckels gemäß einer weiteren Ausgestaltung der Erfindung durch Anpressen der Unterseite eines Randflansches am Schraubdeckel an die Dichtung erzielt.

Vorteilhaft ist zur Erzielung der gasdichten Abdichtung zwischen äußerem Deckel und Behälterrand vorgesehen, daß die miteinander abdichtend zusammenwirkende Dichtkontur und Gegenkontur sich zum Boden des Behälters hin verjüngen und jeweils mehrere in axialer Richtung folgende, ringförmige Vertiefungen und Ausbuchtungen aufweisen, wobei in montiertem Zustand eine Dichtpressung zwischen gegeneinander drückenden Ausbuchtungen erzeugt ist.

Dabei können abwechselnd in axialer Richtung aufeinander folgend eine Ausbuchtung des einen Teiles in eine Vertiefung des anderen Teiles eingreifen und die nächstfolgende Ausbuchtung des einen Teiles gegen eine Ausbuchtung des anderen Teiles mit der Dichtpressung angedrückt sein. Eine konkrete Ausgestaltung zeichnet sich dadurch aus, daß zwei gegeneinander drückende Ausbuchtungen zwischen insgesamt drei in Vertiefungen eingreifenden Ausbuchtungen vorgesehen sind.

Bei einer Ausführung mit Steckdeckel können dieser und die damit zusammernwirkende Behälteröffnung in gleicher oder ähnlicher Weise gestaltet sein wie der äußere Deckel und der Behälterrand, um eine gasdichte, das Austreten von Keimen verhindernde Abdichtung auch an dieser Stelle zu schaffen.

Vorzugsweise sind sämtliche Bestandteile des Behälters einschließlich der beiden Deckel aus Kunststoff hergestellt, z. B. im Spritzgußverfahren.

Der Behälter kann zur Platzersparnis mehreckig, insbesondere viereckig und zum Boden hin sich verjüngend ausgebildet sein, wobei dieser Verjüngung für eine Stapelbarkeit ineinander zur Lagerung und zum Antransport des leeren, vom Deckel noch nicht verschlossenen Behälters sorgt.

Die Erfindung ist im folgenden anhand schematischer Zeichnungen an Ausführungsbeispielen mit weiteren Einzelheiten näher erläutert. Es zeigen:
- Fig. 1: einen Teilschnitt durch den oberen Teil eines Behälters mit Deckel und Innendeckel gemäß der Erfindung in einem Axialschnitt;
- Fig. 2: eine Einzelheit bei II in Figur 1 in größerem Maßstab;
- Fig. 3: eine Seitenansicht des Innendeckels;
- Fig. 4: einen Schnitt entlang der Linie IV-IV in Figur 3;
- Fig. 5: eine Draufsicht auf den Deckel;
- Fig. 6: einen Schnitt entlang der Linie VI-VI in Figur 5;
- Fig. 7: einen Teilschnitt wie Fig. 1 durch eine andere Ausführung und
- Fig. 8: eine Einzelheit bei VIII in Fig. 7 in größerem Maßstab.

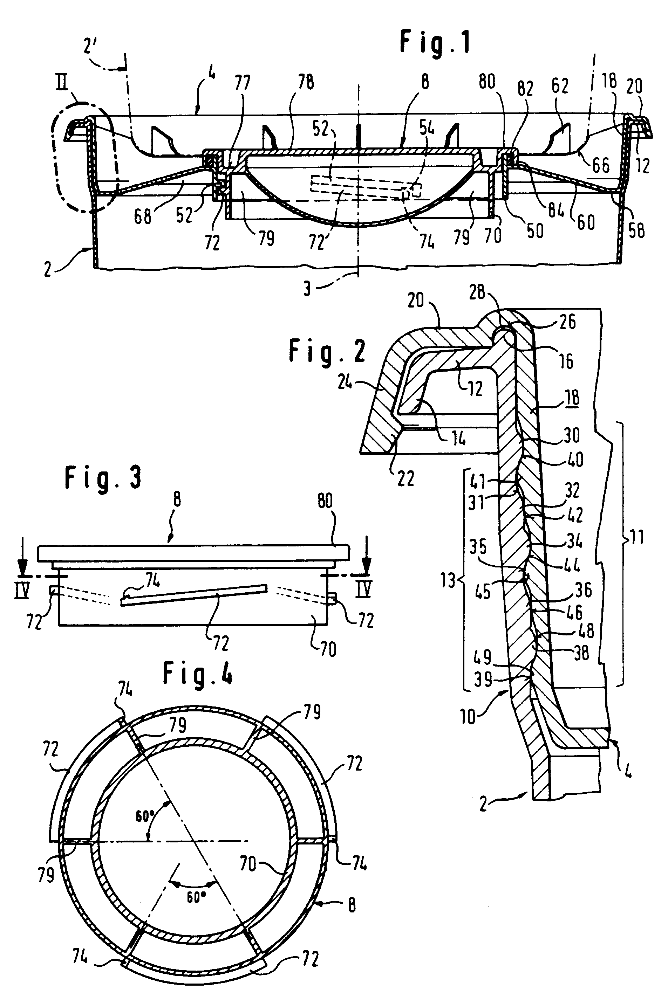

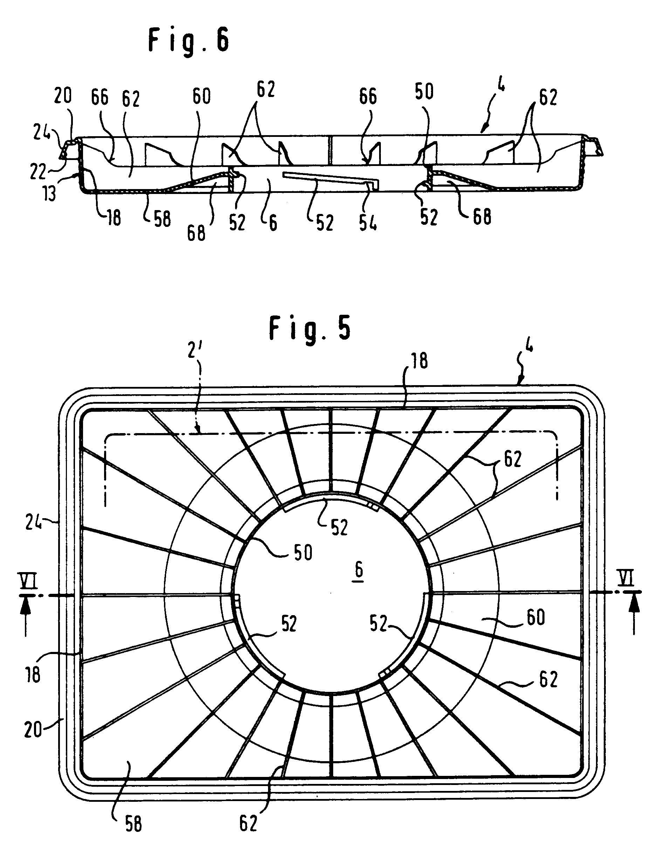

Der in den Figuren 1 und 2 teilweise dargestellte Behälter zeigt einen im Horizontalschnitt rechteckigen Behälterkörper 2 mit parallelen Seitenwänden. In der Draufsicht stellt sich die Außenkontur des Behälters entsprechend der Außenkontur des Deckels nach Figur 5 dar. Die obere Behälteröffnung ist gemäß Figur 1 durch einen Deckel 4 verschlossen. Dieser Deckel 4 hat eine Beschickungsöffnung 6, in welcher ein Innendeckel in Gestalt eines Schraubdeckels 8 eingeschraubt ist. In der Darstellung des Deckels nach Figur 5 und 6 ist der Schraubdeckel aus der Beschickungsöffnung 6 entfernt. Der Behälterkörper 2 verjüngt sich zu seinem Boden hin derart, daß mehrere Behälterkörper bei entferntem Deckel 4 ineinander stapelbar sind. In Figur 1 wie auch in Figur 5 ist mit strichpunktierten Linien der Boden eines gleichartigen Behälters 2' angedeutet.

Anhand der Figur 2 sei nun die Gestaltung der in montiertem Zustand gasdicht zusammenwirkenden Außenbereiche des Behälterkörpers 2 und des Deckels 4 im einzelnen erläutert. Der insgesamt mit der Bezugszahl 10 bezeichnete Behälterrand weist an seinem äußeren, die (nicht bezeichnete) Behälteröffnung begrenzenden Ende ein den Behälterrand einschließendes U-Profil 12 mit einem freien Schenkel 14 auf. An seinem den äußeren Rand der Behälteröffnung bildenden Innenteil hat das U-Profil eine vorspringende Rippe 16.

Ein Außenrandflansch 18 des Deckels 4 verläuft innerhalb und parallel zum Behälterrand 10 in einem leichten Neigungswinkel, z.B. 5°, bezüglich der in Figur 1 mit 3 bezeichneten Achse des Behälterkörpers 2. Der Außenrandflansch 18 weist an seinem oberen Ende ein das U-Profil 12 des Behälterrandes 10 übergreifendes U-Profil 20 mit einem Wulst 22 am Ende des freien Schenkels 24 und einer Nut 26 zur Aufnahme der Rippe 16 auf. Zwischen der Rippe 16 und der Nut 26 ist ein Spalt 28 dargestellt, in welcher eine Dichtmasse eingebracht sein kann.

Die Innenwand des Behälterrandes 10 weist insgesamt fünf axial beabstandete Ausbuchtungen 30, 32, 34, 36, 38 auf. Die mittlere Ausbuchtung 34 und die beiden äußeren Ausbuchtungen 30, 38 ragen weiter nach innen als die dazwischenliegenden Ausbuchtungen 32, 36 und greifen in entsprechende Vertiefungen 40, 44, 48 auf der Außenseite des Außenrandflansches 18 ein. Die dazwischenliegenden Ausbuchtungen 32, 36 liegen mit ihren Scheiteln an den Scheiteln entsprechender Ausbuchtungen 42, 46 des Außenrandflansches 18 unter Dichtpressung an. Diese Dichtpressung ist durch die Dimensionierung des Außenrandflansches mit Übermaß gegenüber der Innenseite des Behälterrandes 10 erzielt und in ihrer Größe durch die elastische Reaktionskraft bestimmt, welche vom Behälterrand 10 auf den Außenrandflansch 18 ausgeübt wird und von dem verwendeten Kunststoff abhängt.

Auf die Vertiefungen 40, 44 und 48 folgen (in axialer Richtung gesehen) jeweils Ausbuchtungen 41, 45 und 49 am Außenrandflansch 18, welche in entsprechende Vertiefungen 31, 35 und 39 auf der Innenseite des Behälterrandes 10 eingreifen.

In der Figur 2 sind die Dichtkontur 30 - 39 am Außenrandflansch 18 zusammenfassend mit 11 und die Gegenkontur 40 - 49 am Behälterrand 10 zusammenfassend mit 13 bezeichnet.

Durch diese in Figur 2 gezeichnete Konfiguration, bei der nicht bezeichnete Spalte jeweils vor und hinter den gegeneinanderdrückenden Ausbuchtungen verbleiben, wird Gasdichtheit erzielt.

Anhand der Figuren 1, 5 und 6 ist nun die weitere Ausgestaltung des Deckels 4 erläutert.

Der Deckel 4 hat einen die Beschickungsöffnung 6 umgrenzenden Ringflansch 50, von dem nach innen drei gleichmäßig über den Umfang verteilte, sich jeweils über 60° erstreckende Gewindeabschnitte 52 ragen. Die Bodenwand 58 des Deckels verläuft ausgehend vom Außenrandflansch 18 zunächst horizontal und hat dann einen leicht bis zum Ringflansch 52 konisch ansteigenden Abschnitt 60. Oberhalb der Bodenwand 58 sind radial zwischen dem Außenrandflansch 18 und dem Ringflansch 50 verlaufende Rippen 62 vorgesehen, die zur Aufnahme des Bodens eines entsprechenden Behälters 2' gestaltete Einbuchtungen 66 haben. Unterhalb des Bodens 58 erstrecken sich ebenfalls radial, jedoch in erheblich geringerer Anzahl Rippen 68 zwischen dem geneigten Abschnitt 60 und dem Ringflansch 50. Die Rippen 62 und 68 sorgen für eine ausreichende Versteifung des Deckels 4 zur Aufnahme des Gewichtes mehrerer darauf gestapelter weiterer Behälter 2'. Bei der in Figur 5 und 6 gezeigten Ausführung sind insgesamt 24, je um 15° versetzte Rippen 62 und insgesamt sechs Rippen 68 radial um die Beschickungsöffnung 6 verteilt angeordnet.

Anhand der Figuren 1, 3 und 4 ist nun die Gestaltung des Schraubdeckels 8 und sein Zusammenwirken mit dem Deckel 4 im einzelnen erläutert. Der Schraubdeckel weist einen nach unten ragenden Ringflansch 70 auf, von dem drei sich jeweils über 60° erstreckende, gleichmäßig am Umfang verteilte Gewindeabschnitte 72 nach außen ragen. Die Gewindeabschnitte 72 sind mit gleicher Neigung, z.B. unter einem Winkel von etwa 5°, wie die Gewindeabschnitte 52 an der Beschickungsöffnung 6 angeordnet und greifen unter diese Gewindeabschnitte 52 in eingeschraubtem Zustand. Figur 1 zeigt den voll eingeschraubten Zustand, bei welchem eine Rastnase 74 an den unteren Enden der Gewindeabschnitte 72 in die Rastaussparungen 54 an den Gewindeabschnitten 52 einrasten. Aus dieser Verrastung ist der Schraubdeckel 8 nicht mehr lösbar. Deshalb wird das aus den Gewindeabschnitten 52, 72 gebildete Gewinde so ausgelegt, daß der Schraubdeckel 8 mit normaler Handkraft nicht in die Raststellung einschraubbar ist, sondern hierzu eine erhöhte Kraft aufgebracht werden muß. Der Schraubdeckel 8 ist über einen Griff 78 drehbar, der über die Oberseite 77 des Schraubdeckels 8 hervorragt und eine für die Handhabung bequeme Breite hat. Der Schraubdeckel hat einen radialen, ringförmigen Randflansch 80, der mit seiner Unterseite gegen eine Dichtung 82 drückt. Diese Dichtung ist zwischen dem oberen Ende des Ringflansches 50 und einem Ringteil 84 aufgenommen, welches am Schraubdeckel 8 oder am Deckel 4 angeformt oder von den beiden Teilen 4, 8 getrennt ausgebildet sein kann. Wichtig ist lediglich, daß die Unterseite des Randflansches 80 zwischen der Dichtung und den sie umgebenden Wandungen eine zur Erzeugung von Gasdichtung ausreichende Dichtpressung im voll eingeschraubten Zustand des Schraubdeckels 8 (siehe Figur 1) erzeugt. Auch der Schraubdeckel 8 ist durch radiale Rippen 79 (Figur 1 und 2) ausgesteift.

Bei einer ausgeführten Konstruktion hat der Behälter ein Volumen von 50 l, eine Höhe von 50 cm und eine Längsbreite von 40 cm und eine Querbreite von 30 cm.

Der Deckel 4 wird bei der erstmaligen Benutzung in der in Figur 1 und 2 gezeigten Form in den oberen Behälterrand gasdicht eingedrückt. Der Schraubdeckel 8 wird bei der darauffolgenden wiederholten Beschickung jedesmal von Hand lediglich soweit eingeschraubt, daß die Rastnase 74 noch nicht in die Rastaussparung 54 einrastet. Das vollständige Einschrauben des Schraubdeckels 8 mit nicht mehr lösbarer Verrasterung der Gewindeabschnitte 52, 72 geschieht erst nach dem letztmaligen Beschickungsvorgang und vor der Entsorgung bzw. einer evtl. Zwischenlagerung.

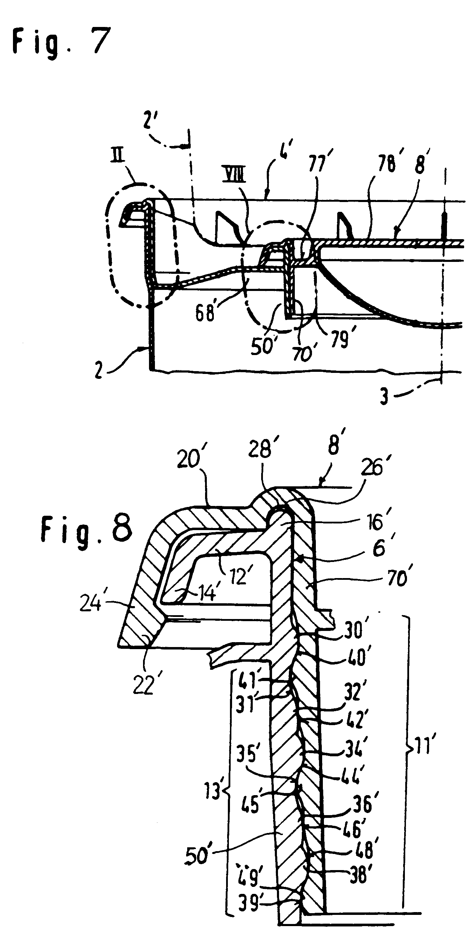

In den Fig. 7 und 8 sind gleiche oder gleichwirkende Teile mit gleichen, jedoch durch einen Strich gekennzeichneten Bezugszeichen versehen. Unterschiedlich zu der Ausführung nach den Fig. 1 bis 6 ist die Ausführung des inneren Deckels als Steckdeckel 8'. Zum Herstellen einer Abdichtung zwischen dem Ringflansch 50' des äußeren Deckels 4' und dem Ringflansch 70' des Steckdeckels 8' ist die gleiche Gestaltung von Dichtkontur 11' und Gegenkontur 13' gewählt, wie sie anhand der Fig. 2 ausführlich beschrieben ist, so daß sich eine nochmalige Beschreibung erübrigt. Bei der Benutzung genügt es, wenn der Steckdeckel 8' nicht nach jedem Einfüllen voll eingesteckt wird; zur Herstellung der endgültigen gasdichten Dichtung sollte der Steckdeckel 8' lediglich nach der letzten Befüllung vollständig in die Lage gemäß Fig. 8 eingedrückt werden.

Mit dem Behälter nach der Erfindung wird eine hygienische Entsorgung von keimbeladenen Gegenständen in einem Krankenhaus ermöglicht, wobei der Behälter sich durch geringen Platzbedarf bei Lagerung und Antransport, durch geringes Gewicht und nach der Befüllung durch Gasdichtheit und Stapelbarkeit übereinander auszeichnet.

## Patentansprüche

1. Einweg-Sammel- und Transportbehälter zur Entsorgung von klinischen Abfall und/oder anderen medizinischen Sondermull mit einer Behälteröffnung mit Dichtkontur (11) und einen entsprechend geformten äußeren Deckel (4; 4') mit Gegenkontur (13), der in die Behälteröffnung eindrückbar ist, wobei Dichtkontur (11) und Gegenkontur (13) abdichtend miteinander zusammenwirken und sich zum Boden des Behälters hin verjüngen, einer Beschickungsöffnung (6; 6) im äußeren Deckel (4; 4') und einen inneren Deckel (8; 8') zum dichten Einbringen in die Beschickungsöffnung (6; 6'),
dadurch **gekennzeichnet,** daß Dichtkontur (11) und Gegenkontur (13) jeweils mehrere, in axialer Richtung folgende, ringförmige Vertiefungen und Ausbuchtungen (30 - 39, 40 - 49) aufweisen, wobei in montiertem Zustand eine Dichtpressung zwischen den gegeneinander drückenden Ausbuchtungen (32, 42, 36, 46) erzeugt ist.

2. Behälter nach Anspruch 1, dadurch **gekennzeichnet,** daß der innere Deckel als Schraubdeckel (8) mit Gewindeabschnitten (72) ausgebildet ist, die unter entsprechende Gewindeabschnitte (52) an einem Ringflansch (50) der Beschickungsöffnung (6) greifen, wobei nach Art einer Bajonett-Verbindung die Abstände zwischen zwei in Umfangsrichtung aufeinander folgenden Gewindeabschnitten (52) am Ringflansch (50) zum Durchlassen der Gewindeabschnitte (72) am Schraubdeckel (8) bemessen sind.

3. Behälter nach Anspruch 2, dadurch **gekennzeichnet,** daß jeweils eine Rastnase (74) am inneren Ende jedes Gewindeabschnittes (72) des Schraubdeckels (8) mit einer Rastaussparung (54) am inneren Ende jedes Gewindeabschnittes (52) am Ringflansch (50) in voll eingeschraubten Zustand des Schraubdeckels (8) zusammenwirkt, und daß in eingeschraubtem Zustand des Schraubdeckels (8) eine gasdichte Anpressung der Unterseite eines Randflansches (80) am Schraubdeckel (8) an eine Dichtung (82) erzielt ist, welche zwischen einem Ringflansch (50) des Deckels (4) und einer ringförmigen Wand (84) aufgenommen ist.

4. Behälter nach Anspruch 1, dadurch **gekennzeichnet**, daß der innere Deckel als Steckdeckel (8') ausgebildet ist und die Beschickungsöffnung (6') eine Dichtkontur (11') und der Steckdeckel (8') eine dazu passende Gegenkontur (13') aufweist, wobei die Dichtkontur (11') und die Gegenkontur (13') gleich wie diejenigen (11, 13) an der Behälteröffnung und am äußeren Deckel (4) ausgebildet sind.

5. Behälter nach Anspruch 1 oder 4, dadurch **gekennzeichnet,** daß die Größe der Dichtpressung zwischen den gegeneinander drückenden Ausbuchtungen (32, 42, 36, 46; 32', 42', 36', 46') durch die Dimensionierung und die Materialelastizität der abdichtend zusammenwirkende Teile definiert ist.

6. Behälter nach Anspruch 1, 4 oder 5, dadurch **gekennzeichnet,** daß abwechselnd in axialer Richtung aufeinander folgend eine Ausbuchtung des einen Teils (18; 70') in eine Vertiefung des anderen Teils (10; 50') eingreift und die nächstfolgende Ausbuchtung des einen Teils gegen eine Ausbuchtung des anderen Teils mit der Dichtpressung angedrückt ist.

7. Behälter nach Anspruch 6, dadurch **gekennzeichnet,** daß zwei gegeneinander drückende Ausbuchtungen (32, 42; 36, 46) zwischen insgesamt drei in Vertiefungen (40, 44, 48) eingreifenden Ausbuchtungen (30, 34, 38) vorgesehen sind.

8. Behälter nach einen der Ansprüche 1 bis 7, dadurch **gekennzeichnet**, daß in eine Nut (26) des äußeren Deckels (4) oder des Steckdeckels (8') eine Rippe (16, 16') am Behälterrand (10) oder an einem die Beschickungsöffnung umgrenzenden Ringflansch (70') des äußeren Deckels (4') eingreift.

9. Behälter nach Anspruch 8, dadurch **gekennzeichnet**, daß ein Spalt (28; 28') zwischen Nut (26; 26') und Rippe (16; 16') mit Dichtmaterial oder einen Dichtring ausgefüllt ist.

10. Behälter nach einen der Ansprüche 1 bis 9, dadurch **gekennzeichnet,** daß der Behälter (2) und der äußere Deckel (4; 4') mehreckig, insbesondere rechteckig und zum Boden hin sich verjüngend ausgebildet sind und aus Kunststoff geformt sind.

11. Behälter nach einem der Ansprüche 1 bis 10, dadurch **gekennzeichnet**, daß der äußere Deckel (4; 4') auf seiner Außenseite radial zur Beschickungsöffnung (6) zwischen dem Ringflansch (50) und dem Außenrandflansch (18) verlaufende Rippen (62) aufweist, die zur Aufnahme des Bodens eines darüber gestapelten gleichartigen Behälters (2') konturiert sind.

## Claims

1. A disposable box for the collection, transport and disposal of clinical waste and/or other special medical waste, the box having: an aperture having a sealing contour (11); a correspondingly shaped outer cover (4; 4') having a companion contour (13) and adapted to be pressed into the aperture, the sealing contour (11) and the companion contour (13) co-operating with one another to provide sealing tightness and narrowing towards the box base; a charging aperture (6; 6) in the outer cover (4; 4'); and an inner cover (8; 8') introducible in sealing-tight manner into the charging aperture (6; 6'), characterised in that the sealing contour (11) and the companion contour (13) are each formed with a number of axially consecutive annular recesses and bulges (30 - 39, 40 - 49), a sealing pressure being produced in the assembled state between the pressed-together bulges (32, 42, 36, 46).

2. A box according to claim 1, characterised in that the inner cover is a screwthreaded cover (8) having screwthreaded parts (72) engaging below companion screwthreaded parts (52) on a ring flange (50) of the charging aperture (6), the intervals which are present between two peripherally consecutive screwthreaded parts (52) of the ring flange (50) and through which the screwthreaded parts (72) of the screwthreaded cover (8) can extend being dimensioned after the fashion of a bayonet connection.

3. A box according to claim 2, characterised in that a click projection (74) at the inner end of each screwthreaded part (72) of the screwthreaded cover (8) co-operates with a click recess (54) at the inner end of each screwthreaded part (52) of the ring flange (50) in the fully screwed-in state of the latter cover (8) and in the screwed-in state thereof the underside of an edge flange (80) of the screwthreaded cover (8) is pressed in gas-tight manner on a seal (82) received between a ring flange (50) of the cover (4) and an annular wall (84).

4. A box according to claim 1, characterised in that the inner cover is a push-in cover (8'), the charging aperture (6') has a sealing contour (11') and the push-in cover (8') has a matching contour (13'), the sealing contour (11') and the matching contour (13') being devised in the same way as those (11, 13) of the box aperture and outer cover (4).

5. A box according to claim 1 or 4, characterised in that the magnitude of the sealing pressure between the pressed-together bulges (32, 42, 36, 46; 32', 42', 36', 46') is defined by the dimensioning and by the resilience of the material of the sealingly co-operating parts.

6. A box according to claims 1, 4 or 5, characterised in that a bulge of one part (18; 70') engages in a recess in the other part (10; 50') in alternate and axially consecutive manner and the immediately following bulge of one part is pressed on a bulge of the other part by the sealing pressure.

7. A box according to claim 6, characterised in that two bulges (32, 42; 36, 46) which press on one another are provided between a total of three bulges (30, 34, 38) engaging in recesses (40, 44, 48).

8. A box according to any of claims 1 to 7, characterised in that a rib (16, 16') on the box edge (10) or on a ring flange (70') of the outer cover (4'), such flange bounding the charging aperture, engages in a groove (26) in the outer cover (4) or push-in cover (8').

9. A box according to claim 8, characterised in that a gap (28; 28') between the groove (26, 26') and the rib (16,; 16') is filled with sealant or a ring seal.

10. A box according to any of claims 1 to 9, characterised in that the box (2) and outer cover (4, 4') are polygonal, more particularly rectangular, narrow towards the base and are moulded from plastics.

11. A box according to any of claims 1 to 10, characterised in that the outer cover (4, 4') has external ribs (62) which extend radially of the charging aperture (6) between the ring flange (50) and the outer edge flange (18) and are contoured to receive the base of a similar box (2') stacked on top.

## Revendications

1. Récipient de collecte et de transport à usage unique pour l'élimination de déchets cliniques et/ou d'autres déchets spéciaux médicaux, comprenant une ouverture de récipient avec contour d'étanchéité (11) et un couvercle extérieur (4; 4') de forme correspondante, avec contour antagoniste (13), que l'on peut enfoncer dans l'ouverture de récipient, le contour d'étanchéité (11) et le contour antagoniste (13) interagissant l'un avec l'autre de façon étanche et se rétrécissent vers le fond du récipient, comprenant une ouverture de remplissage (6; 6*) dans le couvercle extérieur (4; 4') et comprenant un couvercle intérieur (8; 8') pour une mise en place étanche dans l'ouverture de remplissage (6; 6'), caractérisé en ce que le contour d'étanchéité (11) et le contour antagoniste (13) présentent chacun plusieurs creux et indentations (30 - 39, 40 - 49) annulaires qui se suivent dans le sens axial, une pression d'étanchéité étant produite, à l'état monté, entre les indentations (32, 42, 36, 46) se pressant les unes contre les autres.

2. Récipient selon la revendication 1, caractérisé en ce que le couvercle intérieur est réalisé sous la forme d'un couvercle à vis (8) avec tronçons filetés (72) qui, par des tronçons filetés correspondants (52), entrent en prise avec une bride annulaire (50) de l'ouverture de remplissage (6), les écarts séparant deux tronçons filetés (52) se suivant dans le sens circonférentiel sur la bride annulaire (50) étant, à la façon d'un raccordement à baïonnette, dimensionnés pour laisser passer les tronçons filetés (72) sur le couvercle à vis (8).

3. Récipient selon la revendication 2, caractérisé en ce que, à chaque fois, un bec de crantage (74) situé sur l'extrémité intérieure de chaque tronçon fileté (72) du couvercle à vis (8) interagit avec un creux de crantage (54) situé sur l'extrémité intérieure de chaque tronçon fileté (52) sur la bride annulaire (50) quand le couvercle à vis (8) est complètement vissé, et en ce que, quand le couvercle à vis (8) est vissé, il se produit une pression, étanche aux gaz, de la partie inférieure d'une bride de bord (80) située sur le couvercle à vis (8) contre un joint d'étanchéité (82) qui est logé entre une bride annulaire (50) du couvercle (4) et une paroi de forme annulaire (84).

4. Récipient selon la revendication 1, caractérisé en ce que le couvercle intérieur est réalisé sous la forme d'un couvercle enfichable (8') et en ce que l'ouverture de remplissage (6') présente un contour d'étanchéité (11') et le couvercle enfichable (8') présente un contour antagoniste (13') qui lui est adapté, le contour d'étanchéité (11') et le contour antagoniste (13') étant réalisés de la même façon que les éléments (11, 13) sur l'ouverture de récipient et sur le couvercle extérieur (4).

5. Récipient selon la revendication 1 ou 4, caractérisé en ce que l'importance de la pression d'étanchéité entre les indentations (32, 42, 36, 46; 32', 42', 36', 46') qui se pressent les unes contre les autres est définie par le dimensionnement et l'élasticité de matériau des parties interagissant de façon étanche.

6. Récipient selon la revendication 1, 4 ou 5, caractérisé en ce qu'une indentation d'une partie (18; 70') engrène, de façon alternée et consécutive dans le sens axial, dans un creux de l'autre partie (10; 50') et en ce que l'indentation suivante d'une partie est pressée contre une indentation de l'autre partie avec le joint d'étanchéité.

7. Récipient selon la revendication 6, caractérisé en ce que deux indentations (32, 42; 36, 46) se pressant l'une contre l'autre sont prévues entre un total de trois indentations (30, 34, 38) engrenant dans des creux (40, 44, 48).

8. Récipient selon une des revendications 1 à 7, caractérisé en ce que, sur le bord de récipient (10) ou sur une bride annulaire (70') délimitant l'ouverture de remplissage du couvercle extérieur (4'), une nervure (16, 16') engrène dans une rainure (26) du couvercle extérieur (4) ou du couvercle enfichable (8').

9. Récipient selon la revendication 8, caractérisé en ce qu'un interstice (28; 28') entre la rainure (26; 26') et la nervure (16; 16') est rempli de matériau d'étanchéité ou d'une bague d'étanchéité.

10. Récipient selon une des revendications 1 à 9, caractérisé en ce que le récipient (2) et le couvercle extérieur (4; 4') sont réalisés sous forme polygonale, en particulier rectangulaire, se rétrécissant vers le fond et sont réalisés en matière plastique.

11. Récipient selon une des revendications 1 à 10, caractérisé en ce que le couvercle extérieur (4; 4'), sur son côté extérieur et dans le sens radial par rapport à l'ouverture de remplissage (6), présente des nervures (62), situées entre la bride annulaire (50) et la bride de bord extérieur (18), dont le contour est formé de façon à loger le fond d'un récipient (2') de type semblable qui est empilé dessus.
